# EUROPEAN PATENT APPLICATION

(11) **EP 3 315 494 A1**
(43) Date of publication of application: **02.05.2018**
(21) Application number: 17382208.1
(22) Date of filing: 19.04.2017
(51) Int. Cl.: C07D 249/12, A61K 31/4196

(54) **AMORPHOUS FORM OF LESINURAD AND PROCESSES FOR ITS PREPARATION**

(71) Applicant: Química Sintética, S.A., 08028 Barcelona (ES)
(72) Inventor: Barreca, Giuseppe, 23874 MONTEVECCHIA (IT); Ventimiglia, Gianpiero, 72021 FRANCAVILLA FONTANA (IT)
(74) Representative: ZBM Patents - Zea, Barlocci & Markvardsen

(57) **Abstract**

It is described the amorphous form of the compound lesinurad, having the formula reported below:

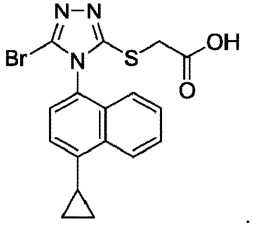

Also described are several processes for the preparation of said amorphous form.

## Description

### Technical Field

The present invention relates to an amorphous form of lesinurad, to a pharmaceutical composition comprising it as well as to the processes for its preparation, and methods of its use.

### Background Art

Lesinurad is the INN denomination assigned to the compound having IUPAC name 2-(5-bromo-4-(4-cyclopropylnaphthalen-1-yl)-4H-1,2,4-triazol-3-ylthio)acetic acid and the formula reported below:

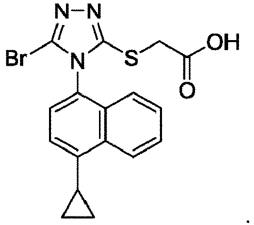

Lesinurad inhibits the uric acid transporter 1 (URAT1) protein, increasing uric acid excretion and thereby lowering serum uric acid, and is used in combination with a xanthine oxidase inhibitor such as allopurinol or febuxostat for the treatment of gout in patients affected by hyperuricemia.

The family of compounds to which lesinurad belongs is disclosed in patent application WO 2006/026356 A2, while patent applications WO 2009/070740 A2, WO 2014/008295 A1 and WO 2014/198241 A1 are directed to processes for the preparation of these compounds.

As generally known, any active principle may exist under amorphous or different crystalline forms (polymorphs), either as pure compound or in forms in which in the structure of the crystal are present molecules of water (hydrates) or of another solvent (solvates); besides, in case of hydrates and solvates, the ratio between the number of molecules of active principle and molecules of water or solvent may vary, giving rise to different solid forms of the compound.

Polymorphism is the ability of a solid material to exist in more than one form or crystal structure. Amorphous solids consist of disordered arrangement of molecules and do not possess a distinguishable crystal lattice. The amorphous form is generally more soluble than the crystalline form and thus contributes more in the bioavailability. An important solid state property of a pharmaceutical compound is its rate of dissolution in aqueous fluid. The rate of dissolution of an active ingredient in a patient's stomach fluid may have therapeutic consequences since it imposes an upper limit on the rate at which an orally-administered pharmaceutical compound may reach the patient's bloodstream. The rate of dissolution is a consideration in formulating syrups, elixirs and other liquid medicaments. The solid state form of a compound may also affect its behavior on compaction and its storage stability.

A new amorphous form of a compound may possess physical properties that differ from, and is advantageous over, those of other crystalline modifications. These include packing properties such as molar volume and density; thermodynamic properties such as glass temperature and solubility; kinetic properties such as dissolution rate; surface properties such as wettability interfacial tension; handling and filtration properties and so on. Variations in any of these properties may affect the chemical and pharmaceutical processing of a compound and may often render the new amorphous form more suitable for pharmaceutical and medical use.

This form diversity has very important implications for the pharmaceutical industry, because each different polymorph, solvate, hydrate or amorphous form may have different properties such as stability, solubility, and hygroscopicity; these differences in physical and chemical behaviors in turn may affect the stability of the formulations in which the active principle is included(possibly requiring the protection from external agents, e.g., temperature, humidity or light), and the bioavailability of the active principle once the formulation is administered to a patient.

For these reasons, chemical compounds useful in the pharmaceutical field are screened looking for the physical form(s) that present the best set of production, storage and handling properties, and which are best suited for administration to the patients.

Patent application WO 2012/092395 A2 discloses two crystalline forms of lesinurad, referred to in the document respectively as Form 1 and Form 2. Form 1 is characterized by a powder X-ray diffraction pattern having the main peaks at about 10.32°, 18.84°, 20.75°, 21.54°, 27.28 2θ, while Form 2 is characterized by a powder X-ray diffraction pattern having the main peaks at about 10.46°, 11.96°, 13.82°, 16.19°, 18.21°, 19.83° 2θ.

Patent application WO 2015/075561 A2 provides crystalline forms of lesinurad, designated as Forms III, IV, V, VI, respectively. Form III is an unsolvated form characterized by a powder X-ray diffraction pattern having the main peaks at about 7.95°, 11.93°, 17.36°, 20.85°, 23.76°, 27.24° 2θ. Form IV is a dichloromethane solvate characterized by a powder X-ray diffraction pattern having the main peaks at about 6.83°, 18.54°, 24.10°, 24.56°, 26.73° 2θ. Form V is a 2-methyl tetrahydrofuran solvate characterized by a powder X-ray diffraction pattern having the main peaks at about 6.12°, 18.70°, 20.10°, 20.88°, 24.84°, 26.21° 2θ. Form VI is a trichloromethane solvate characterized by a powder X-ray diffraction pattern having the main peaks at about 6.64°, 17.94°, 18.27°, 21.28°, 23.47°, 27.68° 2θ.

Patent application CN 104557748 A is directed to two crystalline forms of lesinurad, designated as forms alfa and beta, respectively. Form alfa is characterized by a powder X-ray diffraction pattern having the main peaks at about 6.85°, 11.38°, 18.57°, 24.54°, 26.63° 2θ. Form beta is characterized by a powder X-ray diffraction pattern having the main peaks at about 11.90°, 17.78°, 20.83°, 23.74°, 31.66° 2θ.

The processes described in the prior art report novel crystalline forms of lesinurad. The prior art methods have failed to describe a process for the preparation of a pure amorphous form of lesinurad . Hence, the need was felt to develop process for preparation of the amorphous form of lesinurad.

### Summary of the invention

These objectives are achieved with the present invention that, in a first aspect thereof, relates to an amorphous form of 2-(5-bromo-4-(4-cyclopropylnaphthalen-1-yl)-4H-1,2,4-triazol-3-ylthio)acetic acid (lesinurad), which shows an XRPD profile comprising a halo between 14° and 32° 2θ, when collected with the Kα radiation of copper (λ = 1.5408 Å).

In a second aspect thereof, the invention relates to several processes for producing said amorphous form.

### Brief description of the drawings

Figure 1 represents the XRD powders diffractogram of the amorphous form of lesinurad obtained as per Example 1 of the present invention.
Figures 2 shows the results of DSC test on a sample of amorphous form of lesinurad prepared according to Example 1 of the present invention.

### Detailed description of the invention

All terms used in this application, unless otherwise specified, are to be understood in their ordinary meaning as known in the technical field.

The term "*abouf*" includes the range of experimental errors, which can normally occur performing a measurement.

In its first aspect, the present invention relates to an amorphous form of 2-(5-bromo-4-(4-cyclopropylnaphthalen-1-yl)-4*H*-1,2,4-triazol-3-ylthio)acetic acid (lesinurad), which shows an XRPD profile comprising a halo between 14° and 32° 2θ, when collected with the Kα radiation of copper (λ = 1.5408 Å).

In another aspect, the present invention provides a process for preparing the amorphous form of lesinurad, comprising the steps of:
a) dissolving lesinurad in a solvent or in a mixture of solvents;
b) removing the solvent or a mixture of solvents from the solution obtained in step a) so as to obtain a solid.

In step a), lesinurad is dissolved in a solvent or a mixture of solvents comprising ketones (preferably acetone), alcohols (preferably methanol and ethanol), ethers (e.g. methyl *tert*-butyl ether, 2-methyltetrahydrofuran, diisopropylether, or preferably tetrahydrofuran) or chlorinated solvents (preferably dichloromethane) at temperatures normally between 10 °C and the reflux temperature. The volume of the solvent or mixture thereof normally ranges between 1 mL and 20 mL per gram of lesinurad used; preferably, said volume ranges between 3 and 15 mL per gram of lesinurad, even more preferably 10 mL.

Removal of the solvent or mixture thereof, according to step b), can be carried out with any suitable methods known to the skilled person, e.g. by distillation (at atmospheric or under reduced pressure) or evaporation.

In a possible variant of this embodiment, a further and optional step c) is carried out after step b), comprising drying the solid resulting from step b) when it contains an amount of solvent or solvents above ICH classification (see ICH Topic Q3C (R4) Impurities: Guideline for Residual Solvents, issued by EMEA in February 2009, http://www.ema.europa.eu/docs/en_GB/document_ library/Scientific_guideline/2009/09/WC500002674.pdf).

Said optional step c), can be carried out according to any of the procedures generally known in the field, preferably by treating the solid resulting from step b) at temperatures between 30 and 55 °C (e.g. between 35 and 45 °C, preferably at 40 °C) under reduced pressure.

According to another aspect, the present invention provides a process for preparing amorphous lesinurad, comprising the steps of:
d) dissolving lesinurad in at least one solvent;
e) spray-drying the solution obtained in step d);
f) optionally drying the solid resulting from step e).
   Step d) entails the dissolution of lesinurad in at least one solvent, preferably an organic solvent at temperatures normally between 10 °C and the reflux temperature. The amount of solvent can vary in a very wide range; preferably, the overall volume of liquid may vary between 1 mL and 20 mL per gram of lesinurad; more preferably, the volume is between 3 and 15 mL per gram of lesinurad, even more preferably 10 mL.

Solvents suitable for the purpose are known in the field, such as, for example, a C1-C4 alcohol (for example ethanol, *iso*-propanol or preferably methanol or *tert*-butanol), a C3-C6 ketone (preferably acetone), an ether (preferably tetrahydrofuran) or chlorinated solvents (preferably dichloromethane).

In the subsequent step e) the solution obtained in step d) is spray-dried according to one of the methods generally known in the field.

The term "spray-drying" broadly refers to processes involving breaking-up liquid mixtures into small droplets (atomization) and rapidly removing solvent from the mixtures.

In a typical spray-drying apparatus, the driving force for evaporation of solvent or a mixture of solvents from the droplets is provided by supplying a drying gas. Spray-drying processes and equipment are described, e.g., in Perry's Chemical Engineers Handbook, pp. 2054 to 2057 (6th edition, 1984).

By way of example, the typical spray drying apparatus includes a drying chamber, a source of drying gas that flows into the drying chamber to remove solvent from the atomized-solvent-containing feed, an outlet for the products of drying, and product collection means located downstream of the drying chamber. Typically, the product collection means includes a cyclone connected to the drying apparatus. In the cyclone, the particles produced are separated from the drying gas and evaporated solvent, allowing the particles produced to be collected. A filter can also be used to separate and collect the particles produced by spray-drying. The drying gas used in the invention can be any suitable gas compatible with the substrate, preferably air.

The subsequent step f), can be carried out according to any of the procedures generally known in the field, for example one of those described above to operate step c)

According to another aspect, the present invention provides a process for preparing amorphous lesinurad, comprising the steps of:
g) dissolving lesinurad in a water miscible solvent;
h) precipitating at least a portion of lesinurad in the form of an amorphous solid by mixing the solution obtained in step g) with an antisolvent;
i) recovering the resulting solid and, optionally, drying it.
   Step g) entails the dissolution of lesinurad in at least one water miscible solvent at temperatures normally between 10 °C and the reflux temperature. The amount of solvent can vary in a very wide range; preferably, the overall volume of liquid may vary between 1 mL and 20 mL per gram of lesinurad; more preferably, the volume is between 3 and 15 mL per gram of lesinurad, even more preferably 10 mL.

Solvents suitable for the purpose are known in the field, such as, for example, ketones (preferably acetone), alcohols (preferably methanol, ethanol and 2-propanol), ethers (preferably tetrahydrofuran), dimethylacetamide, dimethylformamide, dimethyl sulfoxide or mixtures thereof.

The following step h) includes mixing the solution of lesinurad prepared in step g) with an antisolvent (preferably water) in order to precipitate at least a portion of lesinurad as an amorphous solid. The antisolvent functions to change the equilibrium solubility of lesinurad in the solution prepared in step g) such that the lesinurad concentration is supersaturated (i.e. above its solubility limit). Because Lesinurad is above its equilibrium solubility limit, it precipitates from the solution in amorphous form. Useful antisolvents are those in which lesinurad is sparingly soluble, such as those in which this compound is soluble in amounts of not more than about 0.1% by weight at about 20-25°C.

The volume ratio between the antisolvent and the water miscible solvent used in step g) typically ranges from 1:5 to 1:50, more typically between 1:8 to 1:13.

According to a preferred embodiment of this aspect of the invention, the solution of lesinurad prepared according to step g) is added to the antisolvent. The direct addition of the antisolvent to the solution of lesinurad prepared in step g) or the inverse addition of said solution to the antisolvent can be carried out in a single step (i.e. a single addition of the entire volume of the solvent or solution to be added) or in multiple additions.

After precipitation, the amorphous lesinurad is recoverd using known techniques such as filtration or centrifugation and optionally dried, e.g. according to the procedures detailed above to operate step c).
lesinurad that can be used in step steps a), d), g) include, for example, polymorphs 1, 2, III, IV, V, VI, alpha, beta described above, or a mixture thereof.

According to another aspect thereof, the present invention provides amorphous lesinurad obtainable according to anyone of the processes described herein.

The instruments and methods used to characterize the amorphous lesinurad prepared according to the invention are as follows:
**XRPD:** Analyses were performed on an EasyX600 TNX bench-top diffractometer at 25 °C, using a Cu Kα tube (30 kV, 20 mA, λ = 1.5408 Å) as the X-ray source, equipped with a scintillation detector. Data collection was made in coupled mode and in theta-theta configuration, with 2θ step of 0.04°. Samples were accurately grinded and placed in the hollow of a spinning aluminum sampler. The instrument was previously calibrated by means of zinc oxide, then allowing collection and elaboration of data by means of DFP acquisition software. RH (relative humidity) in the cabin: 28-30%. Fluorescence was not filtered.
**DSC:** DSC tests were conducted by use of a Mettler-Toledo DSC1 Star^{e} System. Indium was used for calibration. Accurately weighed samples (3-7 mg) were placed in open aluminum pans and heated at a rate of 10 °C/min under a 70 ml/min nitrogen purge. Range from 30 °C up to 300 °C was investigated.

The invention will be further illustrated by the following examples.

### Examples

### Example 1. Preparation of amorphous form of lesinurad.

5 grams of lesinurad were suspended in 50 mL of acetone at 20-25 °C in a glass flask equipped with thermometer, magnetic stirrer and condenser. Under stirring, the suspension was heated up to 45 °C in order to obtain a clear solution. Solvent was removed *in vacuo* by means of a rotary evaporator, thus affording amorphous Lesinurad as a pale-yellow spongy solid.

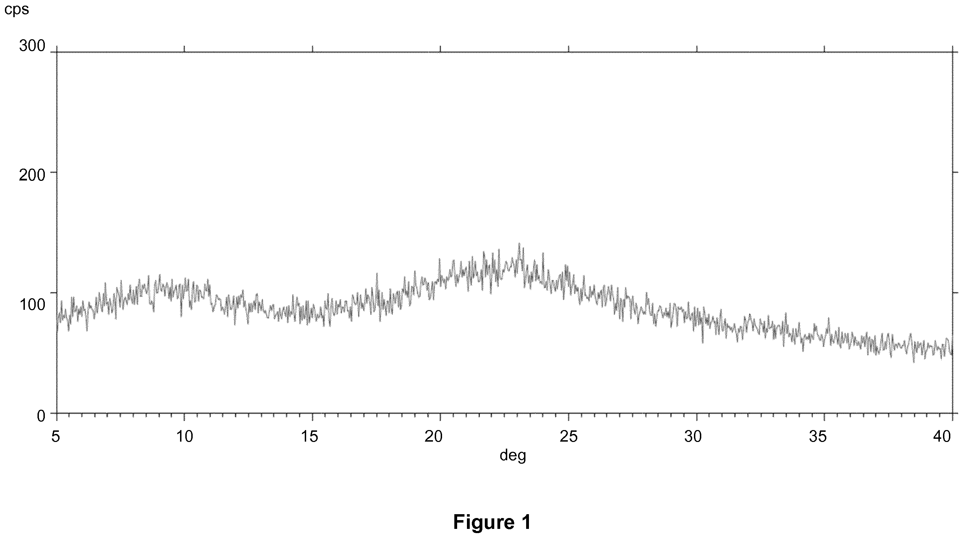

The obtained product was analysed by XRPD, obtaining the diffractogram shown in Figure 1.

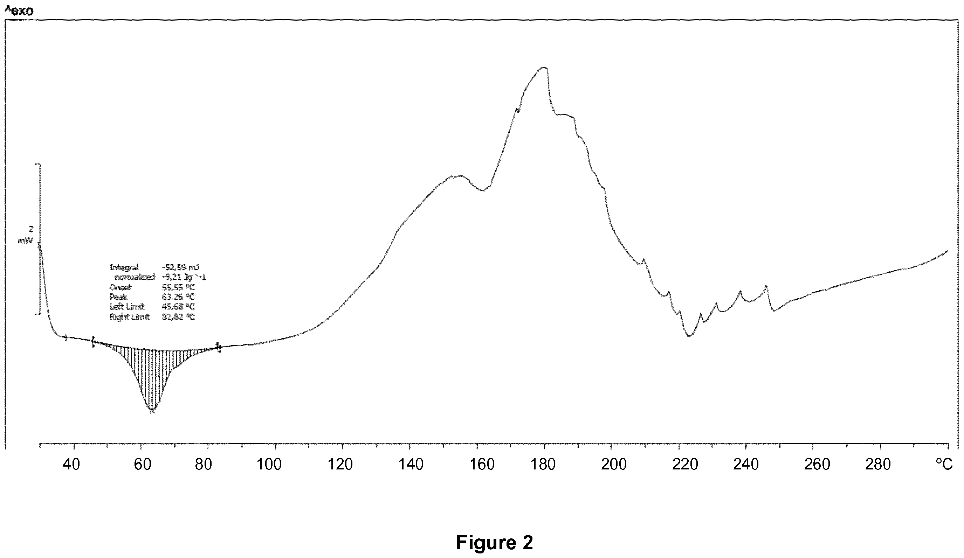

The product was also subjected to DSC analysis, which gave as result the graph shown in Figure 2.

### Example 2. Preparation of amorphous form of lesinurad.

10 grams of lesinurad were suspended in 100 mL of acetone at 20-25 °C in a glass flask equipped with thermometer, magnetic stirrer and condenser. Under stirring, the suspension was heated up to 45 °C in order to obtain a clear solution. The resulting solution was spray-dried by means of a B-290 Mini Spray-Drying Buchi apparatus according to the conditions disclosed in the present invention, thus affording 4.3 grams of amorphous lesinurad as white powder.

The obtained product was analysed by XRPD, obtaining a diffractogram corresponding to the one obtained in example 1.

The product was also subjected to DSC analysis, which gave as result the graph corresponding to the one obtained in example 1.

### Example 3. Preparation of amorphous form of lesinurad.

2 grams of lesinurad were suspended in 7 mL of acetone at 20-25 °C in a glass flask equipped with thermometer, magnetic stirrer and condenser. Under stirring, the suspension was heated up to 45 °C in order to obtain a clear solution. The obtained solution was poured into 260 mL of water at 0 - 5 °C, thus causing lesinurad to precipitate. The obtained solid was filtered and dried up to constant weight upon standing at atmospheric pressure, thus affording 1.9 grams of amorphous lesinurad as white granular solid.

The obtained product was analysed by XRPD, obtaining a diffractogram corresponding to the one obtained in example 1.

The product was also subjected to DSC analysis, which gave as result the graph corresponding to the one obtained in example 1.

### Example 4. Preparation of amorphous form of lesinurad.

2 grams of lesinurad were suspended in 2.5 mL of DMSO at 20-25 °C in a glass flask equipped with thermometer, magnetic stirrer and condenser. The suspension was stirred in order to obtain a clear solution. The obtained solution was poured into 20 mL of water at 0 - 5 °C, thus causing lesinurad to precipitate. The obtained suspension was maintained under stirring at 2 - 5 °C for one additional hour, then the solid was filtered, washed with water and dried at 40 - 45 °C, thus affording 1.8 grams of amorphous lesinurad as white granular solid.

The obtained product was analysed by XRPD, obtaining a diffractogram corresponding to the one obtained in example 1.

The product was also subjected to DSC analysis, which gave as result the graph corresponding to the one obtained in example 1.

### Example 5. Preparation of amorphous form of lesinurad.

One gram of lesinurad was suspended in 3 mL of methanol at 20-25 °C in a glass flask equipped with thermometer, magnetic stirrer and condenser. The suspension was heated up to 40 - 45 °C in order to obtain a clear solution and the obtained solution was poured into 40 mL of water at 0 - 5 °C, thus causing the title compound to precipitate. The obtained suspension was maintained under stirring at 2 - 5 °C for one additional half hour, then the solid was filtered, washed with water and dried at 40 - 45 °C, thus affording 0.9 grams of amorphous lesinurad as white granular solid.

The obtained product was analysed by XRPD, obtaining a diffractogram corresponding to the one obtained in example 1.

The product was also subjected to DSC analysis, which gave as result the graph corresponding to the one obtained in example 1.

## Claims

1. Amorphous lesinurad **characterized by** an XRPD profile comprising a halo between 14° and 32° 2θ.

2. A process for preparing amorphous lesinurad as defined in claim 1, comprising the steps of:
a) dissolving lesinurad in a solvent or in a mixture of solvents;
b) removing the solvent or a mixture of solvents from the solution obtained in step a) so as to obtain a solid.

3. The process according to claim 2, wherein the solvent used in step a) is selected from the group consisting of ketones, alcohols, ethers, chlorinated solvents, and mixtures thereof.

4. The process according to claims 2 or 3, wherein the solvent used in step a) is selected from acetone, methanol, ethanol, methyl *tert*-butyl ether, 2-methyltetrahydrofuran, diisopropylether, tetrahydrofuran, dichloromethane, and mixtures thereof.

5. The process according to any one of claims 2 to 4, wherein step b) is carried out by distillation or evaporation.

6. The process according to any one of claims 2 to 5, further comprising step c) in which the solid resulting from step b) is dried.

7. A process for preparing amorphous lesinurad as defined in claim 1, comprising the steps of:
d) dissolving lesinurad in at least one solvent;
e) spray-drying the solution obtained in step d);
f) optionally drying the solid resulting from step e).

8. The process according to claim 7, wherein the solvent used in step d) is selected from a C1-C4 alcohol, a C3-C6 ketone, an ether, a chlorinated solvent, or a mixture thereof.

9. The process according to claims 7 or 8, wherein the solvent used in step d) is selected from methanol, ethanol, *iso*-propanol, *tert*-butanol, acetone, tetrahydrofuran, and dichloromethane.

10. A process for preparing amorphous lesinurad as defined in claim 1, comprising the steps of:
g) dissolving lesinurad in a water miscible solvent;
h) precipitating at least a portion of lesinurad in the form of an amorphous solid by mixing the solution obtained in step g) with an antisolvent;
i) recovering the resulting solid and, optionally, drying it.

11. The process according to claim 10, wherein the solvent used in step g) is selected from acetone, methanol, ethanol, 2-propanol, tetrahydrofuran, dimethylacetamide, dimethylformamide, dimethyl sulfoxide, and mixtures thereof.

12. The process according to claims 10 or 11, wherein step h) is carried out by addition of the antisolvent to the solution of lesinurad prepared in step g).

13. The process according to any one of claims 10 to 12, wherein the antisolvent used in step h) is a dispersing agent in which lesinurad is soluble in amounts of not more than about 0.1 % by weight at 20-25°C.

14. The process according to any one of claims 10 to 13, wherein the antisolvent used in step h) is water.

15. The process according to any one of claims 2 to 14, wherein steps a), d) or g) are carried out at a temperature between 10 °C and the reflux temperature of the selected solvent or mixture of solvents.
